(19) Europäisches Patentamt
European Patent Office
Office européen des brevets

(11) Numéro de publication : **0 324 691 B1**

# (12) FASCICULE DE BREVET EUROPEEN

(45) Date de publication du fascicule du brevet : **27.12.91 Bulletin 91/52**

(51) Int. Cl.[5] : **C07D 233/94,** C07D 405/04, A61K 31/415

(21) Numéro de dépôt : **89400095.9**

(22) Date de dépôt : **12.01.89**

## (54) Procédé de préparation d'hydroxyalkyl-1 nitro-5 imidazoles.

(30) Priorité : **15.01.88 FR 8800415**

(43) Date de publication de la demande : **19.07.89 Bulletin 89/29**

(45) Mention de la délivrance du brevet : **27.12.91 Bulletin 91/52**

(84) Etats contractants désignés : **AT BE CH DE ES FR GB GR IT LI LU NL SE**

(56) Documents cités :
**EP-A- 0 150 407**
**FR-M- 3 270**
**US-A- 3 178 446**
**US-A- 3 743 653**

(73) Titulaire : **RHONE-POULENC SANTE**
**20, avenue Raymond Aron**
**F-92160 Antony (FR)**

(72) Inventeur : **Buforn, Albert**
**5 rue de Champagneux**
**F-69008 Lyon (FR)**
Inventeur : **Mandard-Cazin, Bernadette**
**32 rue des Ecoles**
**F-94140 Alfortville (FR)**
Inventeur : **Massonneau, Viviane**
**Charrière Blanche Pins 5**
**F-69130 Ecully (FR)**
Inventeur : **Mulhauser, Michel**
**Immeuble "Frênes 4" Résidence Charrière Blanche**
**F-69130 Ecully (FR)**

(74) Mandataire : **Pilard, Jacques et al**
**RHONE-POULENC RECHERCHES Service Brevets Pharma 25, Quai Paul Doumer**
**F-92408 Courbevoie Cedex (FR)**

EP 0 324 691 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention concerne un nouveau procédé de préparation d'hydroxyalkyl-1 nitro-5 imidazole.

Parmi les dérivés de l'imidazole, l'hydroxyéthyl-1 méthyl-2 nitro-5 imidazole (ou métronidazole), l'(hydroxy-2 propyl)-1 méthyl-2 nitro-5 imidazole (ou secnidazole) et l'(hydroxy-3 propyl)-1 méthyl-2 nitro-5 imidazole (ou ternidazole)présentent un intérêt tout particulier du fait de leurs propriétés thérapeutiques remarquables.

Il est connu de préparer le métronidazole par action d'un excès d'oxyde d'éthylène sur le méthyl-2 nitro-4 (ou -5) imidazole dans les conditions décrites dans le brevet français FR 1379915. Cependant les rendements ne sont pas satisfaisants.

Il est connu de préparer le (fluoro-4 phényl)-2 hydroxyéthyl-1 nitro-5 imidazole par action du sulfate d'éthylène sur le (fluoro-4 phényl)-2 nitro-4 (ou -5) imidazole selon le procédé décrit dans le brevet américain US 3743653. Cependant, dans ce cas le rendement est inférieur à 10%.

Il a maintenant été trouvé, et c'est ce qui fait l'objet de la présente invention, que les hydroxyalkyl-1 nitroimidazoles de formule générale :

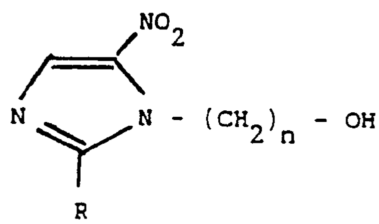

(I)

dans laquelle R représente un atome d'hydrogène ou un radical alkyle contenant 1 à 4 atomes de carbone ou alkényle contenant 2 à 4 atomes de carbone, les radicaux alkyle et alkényle étant éventuellement substitués par un ou plusieurs radicaux, identiques ou différents, choisis parmi les radicaux phényles, phénoxy ou hétérocycliques oxygénés à 5 ou 6 chaînons, ou bien un radical aryle contenant 6 à 10 atomes de carbone éventuellement substitué par un ou plusieurs atomes ou radicaux, identiques ou différents, choisis parmi les atomes d'halogène et les radicaux alkyles contenant 1 à 4 atomes de carbone, alkoxy dont la partie alkyle contient 1 à 4 atomes de carbone, phényle, phénoxy ou nitro, ou bien un radical cycloalkyle contenant 5 à 6 atomes de carbone, les radicaux phényles, phénoxy ou hétérocycliques pouvant être éventuellement substitués par un ou plusieurs atomes ou radicaux, identiques ou différents, choisis parmi les atomes d'halogène et les radicaux alkyles contenant 1 à 4 atomes de carbone, alkoxy dont la partie alkyle contient 1 à 4 atomes de carbone, phényle, phénoxy ou nitro et n est égal à 2 ou 3 et un des atomes de carbone de la chaîne alkylène peut être substitué par un radical méthyle, peuvent être obtenus avec de bons rendements par action d'un sulfate d'alkylène de formule générale :

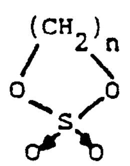

(II)

dans laquelle n est égal à 2 ou 3 et un des atomes de carbone de la chaîne alkylène peut être substitué par un radical méthyle, sur un nitroimidazole de formule générale :

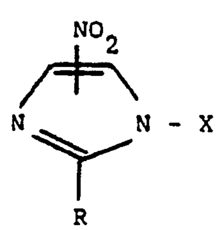

(III)

dans laquelle R est défini comme précédemment et X représente un atome d'hydrogène ou un radical éliminable par hydrolyse ou alcoolyse, tel qu'un radical hydroxyméthyle, alcoxyméthyle dont la partie alcoyle contient 1 à 4 atomes de carbone, acyloxyméthyle dont la partie acyle contient 1 à 4 atomes de carbone ou un radical éthylénique allylique, tel que le radical allyle, ou un radical arylméthyle tel que le radical benzyle, suivie de l'hydrolyse acide ou de l'alcoolyse du produit de condensation ainsi obtenu.

Plus particulièrement la présente invention concerne un procédé de préparation de l'hydroxyéthyl-1 méthyl-2 nitro-5 imidazole, de l'(hydroxy-2 propyl)-1 méthyl-2 nitro-5 imidazole, de l'(hydroxy-3 propyl)-1 méthyl-2 nitro-5 imidazole et de l'hydroxyéthyl-1 nitro-5 imidazole.

Généralement, la condensation du sulfate d'alkylène de formule générale (II) sur le dérivé de l'imidazole de formule générale (III) est effectuée à une température comprise entre 60 et 120°C en présence éventuellement d'un solvant organique choisi parmi les esters tel que l'acétate de méthyle, l'acétate d'éthyle ou le diacétate de glycol, les cétones telles que la méthylisobutylcétone, les éthers tels que le méthyltertiobutyléther, les hydrocarbures aliphatiques ou aromatiques éventuellement halogénés tels que le benzène, le toluène, le xylène, le chloroforme, le dichlorométhane ou le chlorobenzène, ou les nitriles tels que l'acétonitrile.

Le produit de condensation que précipite peut être solubilisé :

— soit dans une solution aqueuse d'un acide minéral fort tel que, par exemple, l'acide sulfurique ou l'acide chlorhydrique

— soit dans un alcool tel que, par exemple, le méthanol ou l'éthanol.

Lorsque le produit de condensation est solubilisé dans l'eau acidifiée, l'hydroxyalkyl-1 nitroimidazole est extrait selon les techniques habituelles après alcalinisation du mélange réactionnel à un pH voisin de 10.

Lorsque le produit de condensation est solubilisé dans un alcool, l'hydroxyalkyl-1 nitroimidazole est isolé selon les techniques habituelles sans traitement préalable du mélange réactionnel.

Pour la mise en oeuvre du procédé, il n'est pas nécessaire d'isoler le produit de condensation intermédiaire, l'hydrolyse ou l'alcoolyse pouvant être enchaînées dans le même appareil.

Le sulfate d'alkylène de formule générale (II), et plus particulièrement le sulfate d'éthylène, peut être obtenu dans les conditions décrites dans le brevet allemand DE 1029382.

Le dérivé du nitroimidazole de formule générale (III) peut être préparé dans les conditions décrites dans le brevet anglais GB 1026631.

Les exemples suivants, donnés à titre non limitatif, montrent comment l'invention peut être mise en pratique.

## EXEMPLE 1

Dans un ballon muni d'un agitateur, on introduit 2 g (0,01 mole) d'acétoxyméthyl-1 méthyl-2 nitro-4 imidazole, 2,6 g (0,02 mole) de sulfate d'éthylène et 5 cm3 de diacétate d'éthylène glycol puis on chauffe pendant 4 heures à 80°C. Le précipité blanc apparu est séparé par filtration et lavé 2 fois par 5 cm3 d'acétate de méthyle. Après séchage, on obtient 3,4 g d'un solide blanc.

L'analyse du filtrat par chromatographie liquide à haute performance (CLHP) montre :

— qu'il contient 98,6 mg d'acétoxyméthyl-1 méthyl-2 nitro-4 imidazole. Le taux de transformation de l'acétoxyméthyl-1 méthyl-2 nitro-4 imidazole est de 95%,

— que le mélange des dérivés imidazolés est constitué de 98,5% d'acétoxyméthyl-1 méthyl-2 nitro-4 imidazole et de 0,5% de méthyl-2 nitro-4 (ou -5) imidazole.

On ajoute 1,705 g du solide blanc obtenu précédemment dans une solution de 1 cm3 d'acide sulfurique concentré dans 5 cm3 d'eau. La solution obtenue est chauffée pendant 10 minutes à 80°C puis est alcalinisée jusqu'à pH = 10 par addition de soude. Le précipité qui se forme est séparé par filtration puis séché. On obtient ainsi 0,685 g d'un produit contenant 95% de métronidazole (dosage par CLHP avec étalonnage interne).

Le rendement en métronidazole isolé est de 76,1% par rapport à l'acétoxyméthyl-1 méthyl-2 nitro-4 imidazole mis en oeuvre.

L'analyse du filtrat montre qu'il contient 69,6 mg de métronidazole et 11,3 mg de méthyl-2 nitro-4 (ou -5) imidazole. Le rendement global en métronidazole est de 84,2% par rapport à l'acétoxyméthyl-1 méthyl-2 nitro-4 imidazole mis en oeuvre et est de 89% par rapport à l'acétoxyméthyl-1 méthyl-2 nitro-4 imidazole transformé.

## EXEMPLE 2

On opère comme dans l'exemple 1 mais en utilisant 2 g d'acétoxyméthyl-1 méthyl-2 nitro-4 imidazole (0,01 mole) et 1,3 g de sulfate d'éthylène (0,01 mole). Après chauffage pendant 4 heures à 80°C, le précipité formé est séparé par filtration et séché. On obtient ainsi 2,85 g d'un produit blanc.

L'analyse du filtrat par CLHP, après alcalinisation à pH = 10, montre qu'il contient 315 mg de méthyl-2 nitro-4 (ou -5) imidazole. Le taux de transformation de l'acétoxyméthyl-1 méthyl-2 nitro-4 imidazole est de 75%.

Le mélange des dérivés de l'imidazole présents dans la solution est constitué de 96% de méthyl-2 nitro-4 (ou -5) imidazole et de 1,5% de métronidazole (dosage par CLHP avec normalisation interne).

On ajoute 0,57 g du produit blanc obtenu précédemment dans 5 cm3 d'éthanol. Le mélange obtenu est chauffé au reflux de l'éthanol pendant 4 heures jusqu'à l'obtention d'une solution homogène.

Après dilution du mélange réactionnel, on dose 201 mg de métronidazole.

Le rendement en métronidazole est de 59% rapport à l'acétoxyméthyl-1 méthyl-2 nitro-4 imidazole mis en oeuvre et est de 81% par rapport à l'acétoxyméthyl-1 méthyl-2 nitro-4 imidazole transformé.

Le mélange des dérivés de l'imidazole présents dans la solution est constitué de 94,8% de métronidazole et de 4,1% de méthyl-2 nitro-4 (ou -5) imidazole.

EXEMPLE 3

On ajoute 0,57 g du produit blanc obtenu à l'exemple 2 dans une solution de 0,2 cm3 d'acide sulfurique concentré dans 2 cm3 d'eau. La solution obtenue est chauffée pendant 1 heure 30 minutes à 80°C puis est alcalinisée jusqu'à pH = 10 par addition de soude.

Dans la solution obtenue, on dose par CLHP, 260 mg de métronidazole.

Le rendement en métronidazole est de 71,6% par rapport à l'acétoxyméthyl-1 méthyl-2 nitro-4 imidazole mis en oeuvre et est de 95% par rapport à l'acétoxyméthyl-1 méthyl-2 nitro-4 imidazole transformé.

Le mélange des dérivés de l'imidazole présents dans la solution est constitué de 92,8% de métronidazole et de 2,5% de méthyl-2 nitro-4 (ou -5) imidazole.

EXEMPLE 4

On opère comme dans l'exemple 2 en utilisant 0,603 g (0,003 mole) d'acétoxyméthyl-1 méthyl-2 nitro-4 imidazole et 0,372 g (0,003 mole) de sulfate d'éthylène dans 3 cm3 de chloroforme. On chauffe pendant 5 heures à la température d'ébullition du chloroforme. Le précipité apparu est séparé par filtration et est lavé avec du chloroforme. Après séchage, on obtient 0,56 g d'un solide blanc.

Dans le filtrat, on dose, par CLHP avec étalonnage externe, 270 mg d'acétoxyméthyl-1 méthyl-2 nitro-4 imidazole et 7,35 mg de méthyl-2 nitro-4 (ou -5) imidazole.

Le mélange des dérivés de l'imidazole dans le filtrat est constitué de 94,6% d'acétoxyméthyl-1 méthyl-2 nitro-4 imidazole et de 3,4% de méthyl-2 nitro-4 (ou -5) imidazole.

Le solide blanc obtenu précédemment (0,56 g) est ajouté fà 6 cm3 d'éthanol. Le mélange est chauffé pendant 4 heures à la température de reflux de l'éthanol. Après dilution du mélange réactionnel, on dose, par CLHP, 193 mg de métronidazole et 16,5 mg de méthyl-2 nitro-4 (ou -5) imidazole.

Le rendement en métronidazole est de 38% par rapport à l'acétoxyméthyl-1 méthyl-2 nitro-4 imidazole mis en oeuvre et est de 76% par rapport à l'acétoxyméthyl-1 méthyl-2 nitro-4 imidazole transformé.

Le taux de transformation de l'acétoxyméthyl-1 méthyl-2 nitro-4 imidazole est de 49%.

Le mélange des dérivés de l'imidazole dans la solution est constitué de 89,1% de métronidazole et de 9,6% de méthyl-2 nitro-4 (ou -5) imidazole.

EXEMPLE 5

On opère comme dans l'exemple 2 mais en utilisant 0,208 g (0,001 mole) d'acétoxyméthyl-1 méthyl-2 nitro-4 imidazole et 0,156 g (0,0012 mole) de sulfate d'éthylène dans 2 cm3 de xylène. On chauffe à 80°C pendant 4 heures. On ajoute alors 3 cm3 d'éthanol et chauffe au reflux pendant 3 heures. Dans la solution ainsi obtenue, on dose 103 mg de métronidazole et 44 mg de méthyl-2 nitro-4 (ou -5) imidazole.

Le mélange des dérivés de l'imidazole dans la solution est constitué de 65,2% de métronidazole et de 33,5% de méthyl-2 nitro-4 (ou -5) imidazole.

Le taux de transformation de l'acétoxyméthyl-1 méthyl-2 nitro-4 imidazole est de 67%.

Le rendement en métronidazole est de 58% par rapport à l'acétoxyméthyl-1 méthyl-2 nitro-4 imidazole mis en oeuvre et est de 87% par rapport à l'acétoxyméthyl-1 méthyl-2 nitro-4 imidazole transformé.

EXEMPLE 6

On opère comme dans l'exemple 5 en utilisant comme solvant la méthylisobutylcétone (2 cm3). Après 4 heures de chauffage à 80°C, un précipité gommeux apparaît. On ajoute 3 cm3 d'éthanol et chauffe au reflux pendant 4 heures.

Dans la solution obtenue, on dose 46,8 mg de métronidazole et 94,9 mg de méthyl-2 nitro-4 (ou -5) imidazole.

Le taux de transformation de l'acétoxyméthyl-1 méthyl-2 nitro-4 imidazole est de 28%.

Le rendement en métronidazole est de 26% par rapport à l'acétoxyméthyl-1 méthyl-2 nitro-4 imidazole mis en oeuvre et est de 93,5% par rapport à l'acétoxyméthyl-1 méthyl-2 nitro-4 imidazole transformé.

EXEMPLE 7

On opère comme dans l'exemple 5 mais en utilisant comme solvant l'acétonitrile (2 cm3). On chauffe à 80°C pendant 4 heures. Un précipité blanc apparaît. On ajoute 3 cm3 d'éthanol puis on chauffe au reflux pendant 4 heures.

Dans la solution, on dose 57,8 mg de métronidazole, 30,2 mg d'acétoxyméthyl-1 méthyl-2 nitro-4 imidazole et 58,3 mg de méthyl-2 nitro-4 (ou -5) imidazole.

Le taux de transformation de l'acétoxyméthyl-1 méthyl-2 nitro-4 imidazole est de 41%.

Le rendement en métronidazole est de 32,5% par rapport à l'acétoxyméthyl-1 méthyl-2 nitro-4 imidazole mis en oeuvre et est de 79% par rapport à l'acétoxyméthyl-1 méthyl-2 nitro-4 imidazole transformé.

EXEMPLE 8

Dans un ballon muni d'une agitation, on introduit 0,573 g (0,00288 mole) d'acétoxyméthyl-1 méthyl-2 nitro-4 imidazole et 0,4 g (0,00322 mole) de sulfate d'éthylène. Le mélange réactionnel est chauffé pendant 1 heure à 90°C. On ajoute alors 5 cm3 d'éthanol puis chauffe au reflux pendant 4 heures.

Après dilution, on dose dans la solution obtenue 393 mg de métronidazole et 23,7 mg de méthyl-2 nitro-4 (ou -5) imidazole.

Le mélange des dérivés de l'imidazole dans la solution est constitué de 6,9% de méthyl-2 nitro-4 (ou -5) imidazole et de 91% de métronidazole.

Le taux de transformation de l'acétoxyméthyl-1 méthyl-2 nitro-4 imidazole est de 93,5%.

Le rendement en métronidazole est de 80% par rapport à l'acétoxyméthyl-1 méthyl-2 nitro-4 imidazole mis en oeuvre et est de 85,3% par rapport à l'acétoxyméthyl-1 méthyl-2 nitro-4 imidazole transformé.

EXEMPLE 9

Dans un ballon muni d'un agitateur, on introduit 130 mg de méthyl-2 nitro-4 (ou -5) imidazole (0,001 mole), 130 mg de sulfate d'éthylène (0,001 mole) et 0,53 cm3 de diacétate de glycol. On chauffe à 120°C pendant 1 heure. Il se forme un précipité. On ajoute 30 microlitres d'acide sulfurique (d = 1,83) puis chauffe le mélange réactionnel pendant 4 heures à 120°C. Après refroidissement, on ajoute une solution de 0,2 cm3 d'acide sulfurique concentré dans 1 cm3 d'eau. On chauffe la solution obtenue à 80°C pendant 1 heure 30 minutes.

Après refroidissement, le mélange réactionnel est dilué. Par chromatographie liquide à haute performance (CLHP), on dose 70 mg de métronidazole et 29 mg de méthyl-2 nitro-4 (ou -5) imidazole.

Le taux de transformation du méthyl-2 nitro-4 (ou -5) imidazole est de 77%.

Le rendement en métronidazole est de 41% par rapport au méthyl-2 nitro-4 (ou -5) imidazole mis en oeuvre et 53,2% par rapport au méthyl-2 nitro-4 (ou -5) imidazole transformé.

EXEMPLE 10

Dans un ballon muni d'un agitateur, on introduit 131 mg de méthyl-2 nitro-4 (ou -5) imidazole (0,001 mole), 130 mg de sulfate d'éthylène (0,001 mole) et 2 cm3 de xylène. On chauffe pendant 4 heures à 80°C. On ajoute alors 5 cm3 d'éthanol puis chauffe au reflux pendant 4 heures.

Dans la solution obtenue, on dose, par CLHP, 25 mg de métronidazole et 100 mg de méthyl-2 nitro-4 (ou -5) imidazole.

Le taux de transformation du méthyl-2 nitro-4 (ou -5) imidazole est de 23%.

Le rendement en métronidazole est de 14% par rapport au méthyl-2 nitro-4 (ou -5) imidazole mis en oeuvre et de 60% par rapport au méthyl-2 nitro-4 (ou -5) imidazole transformé.

EXEMPLE 11

Dans un ballon muni d'un agitateur, on introduit 130 mg de méthyl-2 nitro-4 (ou -5) imidazole (0,001 mole) et 130 mg de sulfate d'éthylène (0,001 mole). On chauffe le mélange réactionnel pendant 4 heures à 90°C. On ajoute alors 5 cm3 d'éthanol puis on chauffe au reflux pendant 4 heures.

Après dilution du mélange réactionnel, on dose, par CLHP, 42,4 mg de métronidazole et 76,2 mg de méthyl-2 nitro-4 (ou -5) imidazole.

Le taux de transformation du méthyl-2 nitro-4 (ou -5) imidazole est de 42%.

Le rendement en métronidazole est de 24% par rapport au méthyl-2 nitro-4 (ou -5) imidazole mis en oeuvre et de 58% par rapport au méthyl-2 nitro-4 (ou -5) imidazole transformé.

EXEMPLE 12

Dans un ballon muni d'une agitation, on introduit 12 g (0,06 mole) d'acétoxyméthyl-1 méthyl-2 nitro-4 imidazole, 10 g (0,072 mole) de sulfate de propylène et 40 cm3 de xylène.

Le mélange réactionnel est chauffé à 110°C pendant 5 heures. Dès le début du chauffage un precipité gommeux apparaît. On ajoute alors 20 cm3 d'eau et 1,5 cm3 d'acide sulfurique concentré (0,028 mole) et chauffe au reflux pendant 4 heures.

Le mélange est refroidi à 20°C. La phase xylénique séparée est extraite par 90 cm3 d'eau. Dans les phases aqueuses réunies, on dose par CLHP, 4,32 g de secnidazole, 0,33 g d'(hydroxy-1 méthyl-2) éthyl-1 méthyl-2 nitro-5 imidazole et 3,1 g de méthyl-2 nitro-4 (ou -5) imidazole.

Le taux de transformation de l'acétoxyméthyl-1 méthyl-2 nitro-4 imidazole est de 59%.

Le rendement en secnidazole est de 39% par rapport à l'acétoxyméthyl-1 méthyl-2 nitro-4 imidazole mis en oeuvre et de 64% par rapport à l'acétoxyméthyl-1 méthyl-2 nitro-4 imidazole transformé.

EXEMPLE 13

Dans un ballon muni d'une agitation, on introduit 4,65 g (0,025 mole) d'acétoxyméthyl-1 nitro-4 imidazole, 4,16 g (0,0325 mole) de sulfate d'éthylène et 30 cm3 de xylène. Le mélange est chauffé pendant 6 heures à 80°C. On ajoute alors 30 cm3 d'eau et 2 cm3 d'acide sulfurique concentré. Le mélange réactionnel biphasique est chauffé au reflux pendant 4 heures.

Le dosage de la phase aqueuse par chromatographie liquide à haute performance (CLHP) avec étalonnage externe montre que :

— le taux de transformation de l'acétoxyméthyl-1 nitro-4 imidazole est de 86%

— le rendement en hydroxyéthyl-1 nitro-5 imidazole est de 73,7% par rapport à l'acétoxyméthyl-nitro-4 imidazole transformé.

EXEMPLE 14

Dans un ballon muni d'une agitation, on introduit 2 g (0,01 mole) d'acétoxyméthyl-1 méthyl-2 nitro-4 imidazole, 1,8 g (0,013 mole) de sulfate de propylène et 10 g de xylène. Le mélange est chauffé pendant 6 heures à 100°C. On ajoute alors 10 cm3 d'eau et 1 cm3 d'acide sulfurique concentré. Le mélange réactionnel est chauffé au reflux pendant 3 heures.

Le dosage de la phase aqueuse par chromatographie liquide à haute performance (CLHP) avec étalonnage externe montre que :

— le taux de transformation de l'acétoxyméthyl-1 méthyl-2 nitro-4 imidazole est de 43%

— le rendement en (hydroxy-3 propyl)-1 méthyl-2 nitro-5 imidazole est de 95% par rapport à l'acétoxyméthyl-1 méthyl-2 nitro-4 imidazole transformé.

**Revendications**

1. Procédé de préparation d'hydroxyalkyl-1 nitroimidazoles de formule générale :

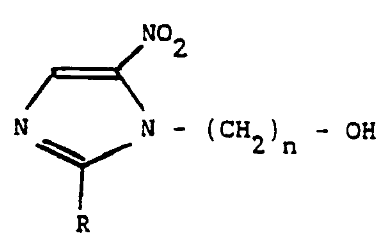

dans laquelle R représente un atome d'hydrogène ou un radical alkyle contenant 1 à 4 atomes de carbone ou alkényle contenant 2 à 4 atomes de carbone, les radicaux alkyle et alkényle étant éventuellement substitués par un ou plusieurs radicaux, identiques ou différents, choisis parmi les radicaux phényles, phénoxy ou hétérocycliques oxygénés à 5 ou 6 chaînons, ou bien un radical aryle contenant 6 à 10 atomes de carbone éventuellement substitué par un ou plusieurs atomes ou radicaux, identiques ou différents, choisis parmi les atomes d'halogène et les radicaux alkyles contenant 1 à 4 atomes de carbone, alkoxy dont la partie alkyle contient 1 à 4 atomes de carbone, phényle, phénoxy ou nitro, ou bien un radical cycloalkyle contenant 5 ou 6 atomes de carbone, les radicaux phényles, phénoxy ou hétérocycliques pouvant être éventuellement substitués par un ou plusieurs atomes ou radicaux, identiques ou différents, choisis parmi les atomes d'halogène et les radicaux alkyles contenant 1 à 4 atomes de carbone, alkoxy dont la partie alkyle contient 1 à 4 atomes de carbone, phényle, phénoxy ou nitro et n est égal à 2 ou 3 et un des atomes de carbone de la chaîne alkylène peut être substitué par un radical méthyle, caractérisé en ce que l'on fait réagir un sulfate d'alkylène de formule générale :

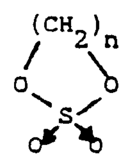

dans laquelle n est égal à 2 ou 3 et un des atomes de carbone de la chaîne alkylène peut être substitué par un radical méthyle, sur un nitroimidazole de formule générale :

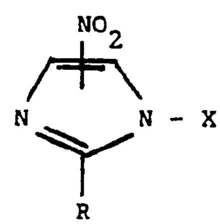

dans laquelle R est défini comme précédemment et X représente un atome d'hydrogène ou un radical éliminable par hydrolyse ou alcoolyse, tel que le radical hydroxyméthyle, alcoxyméthyle dont la partie alcoyle contient 1 à 4 atomes de carbone ou acyloxyméthyle dont la partie acyle contient 1 à 4 atomes de carbone ou un radical éthylénique allylique ou un radical arylméthyle, à une température comprise entre 60 et 120°C en opérant éventuellement dans un solvant organique choisi parmi les esters, les éthers, les cétones, les hydrocarbures aliphatiques ou aromatiques éventuellement halogénés et les nitriles, puis hydrolyse ou alcoolyse le produit ainsi obtenu et isole l'hydroxyalkyl-1 nitro imidazole.

2. Procédé selon la revendication 1 caractérisé en ce que le solvant est choisi parmi l'acétate de méthyle, l'acétate d'éthyle, le diacétate de glycol, les méthyltertiobutyléther, la méthylisobutylcétone, le chloroforme, le dichlorométhane, le benzène, le toluène, le xylène, le chlorobenzène et l'acétonitrile.

3. Procédé selon la revendication 1 caractérisé en ce que l'hydrolyse du produit de condensation est effectuée en présence d'un acide fort choisi parmi l'acide sulfurique et l'acide chlorhydrique.

4. Procédé selon la revendication 1 caractérisé en ce que l'alcoolyse est effectuée par chauffage en présence d'un alcool choisi parmi le méthanol et l'éthanol.

5. Procédé selon la revendication 1 caractérisé en ce que l'hydroxyalkyl-1 nitroimidazole obtenu est le métronidazole, le secnidazole ou le ternidazole ou l'hydroxyéthyl-1 nitro-5 imidazole.

**Patentansprüche**

1. Verfahren zur Herstellung von 1-Hydroxyalkyl-nitroimidazolen der allgemeinen Formel :

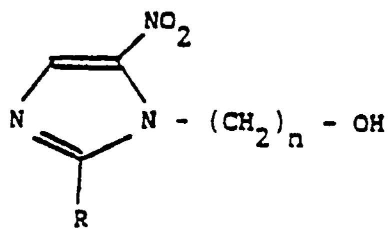

in welcher R ein Wasserstoffatom oder einen Alkylrest mit 1 bis 4 Kohlenstoffatomen oder Alkenylrest mit 2 bis 4 Kohlenstoffatomen, wobei die Alkyl- und Alkenylreste gegebenenfalls durch einen oder mehrere gleiche oder verschiedene Reste, ausgewählt aus den Phenyl-, Phenoxyresten oder sauerstoffhaltigen heterocyclischen Resten mit 5 oder 6 Gliedern, substituiert sind, oder einen Arylrest mit 6 bis 10 Kohlenstoffatomen, gegebenenfalls substituiert durch ein oder mehrere gleiche oder verschiedene Atome oder Reste, ausgewählt aus den Halogenatomen und den Alkylresten mit 1 bis 4 Kohlenstoffatomen, den Alkoxyresten, deren Alkylteil 1 bis 4 Kohlenstoffatome enthält, Phenyl-, Phenoxy- oder Nitroresten, oder einen Cycloalkylrest mit 5 oder 6 Kohlenstoffatomen darstellt, wobei die Phenyl-, Phenoxy- oder heterocyclischen Reste gegebenenfalls durch ein oder mehrere gleiche oder verschiedene Atome oder Reste substituiert sein können, ausgewählt aus den Halogenatomen und den Alkylresten mit 1 bis 4 Kohlenstoffatomen, Alkoxyresten, deren Alkylteil 1 bis 4 Kohlenstoffatome enthält, Phenyl-, Phenoxy- oder Nitroresten, und n gleich 2 oder 3 ist und eines der Kohlenstoffatome der Alkylenkette durch einen Methylrest substituiert sein kann, dadurch gekennzeichnet, daß man ein Alkylensulfat der allgemeinen Formel :

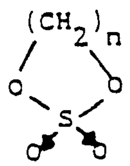

in welcher n gleich 2 oder 3 ist und eines der Kohlenstoffatome der Alkylenkette durch einen Methylrest substituiert sein kann, mit einem Nitroimidazol der allgemeinen Formel :

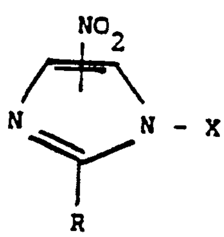

in welcher R die obige Bedeutung hat und X darstellt : ein Wasserstoffatom oder einen durch Hydrolyse oder Alkoholyse entfernbaren Rest, z.B. den Hydroxymethylrest, Alkoxymethylrest, dessen Alkylteil 1 bis 4 Kohlenstoffatome enthält, oder Acyloxymethylrest, dessen Acylteil 1 bis 4 Kohlenstoffatome enthält, oder einen Allyläthylenrest oder einen Arylmethylrest, bei einer Temperatur zwischen 60 und 120°C umsetzt, indem man gegebenenfalls in einem organischen Lösungsmittel, ausgewählt aus den Estern, den Äthern, den Ketonen, den gegebenenfalls halogenierten aliphatischen oder aromatischen Kohlenwasserstoffen, und den Nitrilen, arbeitet, und dann das so erhaltene Produkt hydrolysiert oder alkoholysiert und das 1-Hydroxyalkyl-nitroimidazol isoliert.

2. Verfahren nach Anspruch 1, dadurch gekennzeichnet, daß das Lösungsmittel ausgewählt ist aus Methylacetat, Athylacetat, Glykoldiacetat, Methyl-tert.butyläther, Methylisobutylketon, Chloroform, Dichlormethan, Benzol, Toluol, Xylol, Chlorbenzol und Acetonitril.

3. Verfahren nach Anspruch 1, dadurch gekennzeichnet, daß man die Hydrolyse des Kondensationsprodukts in Gegenwart einer starken Säure, ausgewählt aus Schwefelsäure und Chlorwasserstoffsäure, ausführt.

4. Verfahren nach Anspruch 1, dadurch gekennzeichnet, daß die Alkoholyse ausgeführt wird durch Erhitzen in Gegenwart eines Alkohols, ausgewählt aus Methanol und Äthanol.

5. Verfahren nach Anspruch 1, dadurch gekennzeichnet, daß das erhaltene 1-Hydroxyalkyl-nitroimidazol

Metronidazol, Secnidazol oder Ternidazol oder 1-Hydroxyäthyl-5-nitro-imidazol ist.

## Claims

1. A process for preparing 1-(hydroxyalkyl)-nitroimidazoles of general formula :

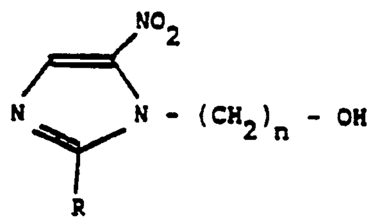

in which R denotes a hydrogen atom or an alkyl radical containing 1 to 4 carbon atoms or alkenyl radical containing 2 to 4 carbon atoms, the alkyl and alkenyl radicals optionally being substituted with one or more identical or different radicals chosen from phenyl, phenoxy or 5- or 6-membered oxygen-containing heterocyclic radicals, or alternatively an aryl radical containing 6 to 10 carbon atoms and optionally substituted with one or more identical or different atoms or radicals chosen from halogen atoms and alkyl radicals containing 1 to 4 carbon atoms, alkoxy radicals in which the alkyl portion contains 1 to 4 carbon atoms, phenyl, phenoxy or nitro radicals, or alternatively a cycloalkyl radical containing 5 to 6 carbon atoms, it being possible for the phenyl, phenoxy or heterocyclic radicals to be optionally substituted with one or more identical or different atoms or radicals chosen from halogen atoms and alkyl radicals containing 1 to 4 carbon atoms, alkoxy radicals in which the alkyl portion contains 1 to 4 carbon atoms, phenyl, phenoxy or nitro radicals, and n is equal to 2 or 3 and one of the carbon atoms of the alkylene chain can be substituted with a methyl radical, wherein an alkylene sulphate of general formula :

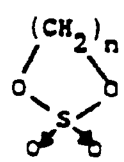

in which n is equal to 2 or 3 and one of the carbon atoms of the alkylene chain can be substituted with a methyl radical, is reacted with a nitroimidazole of general formula :

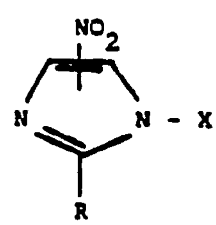

in which R is defined as above and X denotes a hydrogen atom or a radical that can be removed by hydrolysis or alcoholysis, such as a hydroxymethyl radical, alkoxymethyl radical in which the alkyl portion contains 1 to 4 carbon atoms or acyloxymethyl radical in which the acyl portion contains 1 to 4 carbon atoms, or an allylic ethylenic radical or an arylmethyl radical, at a temperature of between 60 and 120°C, optionally working in an organic solvent chosen from esters, ethers, ketones, aliphatic or aromatic hydrocarbons, optionally halogenated, and nitriles, the product thereby obtained is then hydrolysed or alcoholysed and the 1-(hydroxyalkyl)nitroimidazole is isolated.

2. The process according to claim 1, wherein the solvent is chosen from methyl acetate, ethyl acetate, glycol diacetate, methyl tert-butyl ether, methyl isobutyl ketone, chloroform, dichloromethane, benzene, toluene, xylene, chlorobenzene and acetonitrile.

3. The process according to claim 11, wherein the hydrolysis of the condensation product is performed in the presence of a strong acid chosen from sulphuric acid and hydrochloric acid.

4. The process according to claim 1, wherein the alcoholysis is performed by heating in the presence of an alcohol chosen from methanol and ethanol.

5. The process according to claim 1, wherein the 1-(hydroxyalkyl)nitroimidazole obtained is metronidazole, secnidazole, ternidazole or 1-hydroxyethyl-5-nitroimidazole.